# FASCICULE DE BREVET EUROPEEN

(11) **EP 0 694 524 B1**
(45) Date de publication et mention de la délivrance du brevet: **15.10.1997**
(21) Numéro de dépôt: 95401765.3
(22) Date de dépôt: 25.07.1995
(51) Int. Cl.: C07C 67/08, C07C 69/54

(54) **Procédé de fabrication de l'acrylate de butyle par estérification directe**
Verfahren zum Herstellen von Butylacrylat durch direkte Veresterung
Process for the preparation of butyle acrylate by direct esterification

(30) Priorité: 28.07.1994 FR 9409371
(43) Date de publication de la demande: 31.01.1996
(73) Titulaire: ELF ATOCHEM S.A., 92800 Puteaux (FR)
(72) Inventeur: Fauconet, Michel, F-57730 Valmont (FR); Lacroix, Christian, F-57600 Folkling (FR); Hess, Nathalie, F-57500 Saint-Avold (FR); Bessalem, Jacqueline, F-57500 Saint-Avold (FR)
(74) Mandataire: Chaillot, Geneviève

(56) Documents cités:
- EP-A- 0 609 127
- US-A- 3 882 167
- US-A- 5 093 520

## Description

La présente invention porte sur un procédé de fabrication de l'acrylate de butyle par estérification directe de l'acide acrylique par le butanol, ladite estérification étant catalysée par l'acide sulfurique, l'eau de réaction étant éliminée, pendant toute ou partie de la réaction, sous la forme d'un mélange azéotropique avec le butanol et l'acrylate de butyle, le mélange réactionnel brut obtenu comprenant de l'acrylate de butyle, du butanol, de l'acide acrylique, du sulfate acide de butyle et des traces d'acide sulfurique.

La synthèse des acrylates par estérification de l'acide acrylique et, en particulier, de l'acrylate de butyle, a été largement décrite dans la littérature.

La réaction d'estérification est une réaction équilibrée, en général catalysée par un acide. Deux grandes voies de catalyse sont principalement décrites :
- la catalyse homogène, le plus souvent en présence d'acide sulfurique ou d'acides sulfoniques, la réaction étant conduite en discontinu ou en continu ;
- la catalyse hétérogène, à base de catalyseurs solides généralement du type résines acides et une réaction conduite de façon continue.

Dans chacun des cas, l'eau générée pendant la réaction peut être éliminée simultanément à sa formation ou séparée dans une deuxième étape. L'élimination de cette eau est, en général, réalisée par distillation, sous forme d'un mélange azéotropique avec l'alcool estérifiant ou un solvant ajouté avant réaction.

Pour des raisons d'augmentation de productivité et de facilité de conduite de la réaction, il peut être préférable d'opérer en continu. Toutefois, cette voie présente l'inconvénient, si on ne désire pas augmenter indéfiniment le nombre d'étages réactionnels, de limiter le taux de conversion des réactifs.

En particulier, dans le cas de la fabrication de l'acrylate de butyle, l'acide acrylique et l'ester possèdent des points d'ébullition très voisins, et il est économiquement très difficile de séparer les deux composants par une distillation ordinaire.

L'utilisation d'une catalyse de type hétérogène présente l'avantage de faciliter la séparation et le recyclage du catalyseur.

Ce type de catalyse a été abondamment décrit, comme dans le certificat d'addition français n° 2 186 457, dans lequel on élimine l'eau de réaction au fur et à mesure de sa formation, pour favoriser la conversion des réactifs. Ce procédé, utilisant des résines acides comme catalyseurs, présente le désavantage de générer des sous-produits de type éthers de dialkyle, en quantités plus importantes que la catalyse homogène par l'acide sulfurique. En particulier, dans le cas de la fabrication de l'acrylate de butyle, on génère l'éther de dibutyle qui est difficilement séparable de l'acrylate de butyle par distillation. Cette formation d'éther est en outre favorisée dans ce milieu par l'absence d'eau et la présence d'une quantité importante de butanol.

Pour réduire cet inconvénient, lié à l'utilisation de catalyseurs résines acides, il a été proposé, dans le brevet américain US-A-4 012 439, de réaliser l'étape de réaction sans éliminer simultanément l'eau de réaction. Ceci nécessite de travailler sous pression pour maintenir les constituants du mélange à l'état liquide. En outre, cette façon d'opérer réduit la cinétique et le taux de conversion de l'acide acrylique, ce qui impose de travailler à des températures élevées et en présence d'un excès d'alcool élevé. La conséquence est que le milieu réactionnel contient des concentrations importantes d'eau, de butanol et d'acide acrylique, ce qui favorise, sous l'effet de la température, la formation de produits lourds issus de réactions d'addition d'eau, de butanol et d'acide acrylique sur les doubles liaisons de l'acide acrylique et de l'acrylate de butyle. La formation de ces impuretés lourdes conduit en outre à une réduction de l'activité et de la durée de vie du catalyseur par encrassement des pores actifs de la résine.

L'utilisation de la catalyse homogène, en particulier de l'acide sulfurique, réduit fortement ces inconvénients liés à la formation de sous-produits indésirables, mais pose le problème de la séparation du catalyseur après réaction, qui s'ajoute au souci d'éliminer l'acide acrylique résiduel, pour éviter une pollution de l'acrylate de butyle pur par ce réactif non converti.

L'acide sulfurique est souvent préféré, principalement en raison de son activité et de son faible coût, qui rend économiquement viable un procédé d'élimination sans recyclage.

Ainsi, il est décrit d'éliminer conjointement l'acide acrylique et le catalyseur par une neutralisation alcaline de leurs acidités, par exemple en présence de soude caustique.

Cette technique, utilisée simplement, présente des inconvénients importants :
- dans le milieu réactionnel, l'acide sulfurique réagit avec le butanol et se trouve présent sous la forme de sulfate acide de butyle, qui est le véritable catalyseur de la réaction. Lors de l'étape de neutralisation alcaline par la soude, ce composé est transformé en sulfate neutre de butyle, non distillable dans une étape ultérieure de distillation des eaux usées. Par conséquent, on retrouve ce composé dans les eaux épuisées rejetées par l'atelier, ce qui génère une pollution organique importante, mesurable sous forme de DCO ;
- l'acide acrylique en excès, qui n'a pas réagi pendant la réaction, constitue également une source importante de pollution organique, rejetée sous forme de sel alcalin (acrylate de sodium) dans les eaux épuisées de l'atelier. En outre, cette élimination conduit à une perte de rendement de l'acide acrylique en acrylate de butyle.

Pour réduire la pollution organique générée par le rejet du sulfate neutre de butyle dans les eaux épuisées, il est décrit dans la demande de brevet EP 0 609 127 revendiquant la priorité de la demande de brevet français n° 93-00827 du 27 janvier 1993, au nom de la Société déposante, un procédé visant à hydrolyser le sulfate acide de butyle en milieu acide, pour le rétrograder en butanol et acide sulfurique originel éliminable après neutralisation sans augmentation de la pollution organique aqueuse. Ce traitement présente les inconvénients de nécessiter un ajout important d'acide sulfurique, pour neutraliser toutes les espèces alcalines présentes à l'issue de l'étape de neutralisation (acrylate de sodium et sulfate neutre de butyle) et pour catalyser la réaction d'hydrolyse, ce qui a pour conséquence un rejet important de sels alcalins après nouvelle neutralisation des espèces acides.

Le procédé de séparation de l'acide acrylique du milieu réactionnel d'une synthèse d'acrylate de butyle, décrit dans le brevet américain US-A-4 012 439, dans le cas d'une estérification catalysée par résines acides, n'est pas applicable simplement dans le cas d'une estérification catalysée par l'acide sulfurique, car la séparation des constituants légers (acrylate de butyle, butanol, eau) des composés lourds (catalyseur, acide acrylique, inhibiteurs, etc.) nécessiterait une distillation en condition très corrosive, en raison de la présence du catalyseur dans la colonne et en pied de colonne, donc le recours à des matériaux spéciaux coûteux. En outre, la présence de catalyseur à des concentrations élevées dans la colonne, en présence d'eau, conduit à une hydrolyse partielle de l'acrylate de butyle formé, d'où une réduction du rendement de la synthèse.

La récupération simultanée de l'acide acrylique et du catalyseur est décrite dans le brevet américain US-A-5 093 520 dans le cas d'une synthèse d'acrylate de 2-éthyl hexyle, utilisant deux étapes complémentaires :
- extraction à l'eau de l'acide acrylique et du sulfate acide de 2-éthyl hexyle présents dans le milieu réactionnel ;
- extraction à l'alcool 2-éthyl hexanol de l'acide acrylique et du sulfate acide de 2-éthyl hexyle présents dans la phase aqueuse de la première étape d'extraction.

Le procédé nécessite l'utilisation d'un solvant, ajouté à l'étape réactionnelle, notamment pour favoriser le rendement d'extraction des composés dans la première étape d'extraction. L'utilisation d'un solvant nécessite une étape de séparation et de récupération qui est coûteuse. De plus, même dans ces conditions, les rendements d'extraction sont faibles, surtout en ce qui concerne l'acide acrylique. Ce procédé n'est pas applicable à la synthèse de l'acrylate de butyle, car l'acide acrylique non extrait est très difficilement séparable de l'ester. D'autre part, le rendement d'extraction du sulfate acide de butyle par le butanol serait moins bon que pour l'extraction du sulfate acide de 2-éthyl hexyle par le 2-éthyl hexanol, à cause de la plus forte solubilité de l'alcool dans l'eau dans le cas du butanol, ce qui a pour effet d'entraîner une partie du catalyseur dans la phase aqueuse. La conséquence serait le rejet d'une pollution organique importante dans les eaux épuisées de l'atelier.

Le brevet américain US-A-3 882 167 décrit un procédé de synthèse d'esters acryliques avec catalyse hétérogène par des résines acides, dans lequel sont décrites :
- la récupération de l'acide acrylique dans le milieu réactionnel dans une première étape d'extraction alcaline ;
- une réacidification du sel d'acide acrylique ;
- une deuxième étape d'extraction de l'acide acrylique précédemment régénéré à l'aide d'un mélange de légers récupéré en tête de colonne d'étêtage du milieu réactionnel, principalement constitué d'alcool, ester, éther de dialkyle et eau.

Ce procédé présente le désavantage de constituer une boucle de concentration croissante d'impuretés légères, en particulier l'éther, qui finit par sortir dans le produit pur.

En outre, comme dans le cas du brevet américain US-A-5 093 520, il ne serait pas applicable dans le cas de la synthèse de l'acrylate de butyle en catalyse homogène, à cause du rendement d'extraction imparfait du sulfate acide de butyle par le butanol.

Le brevet américain US-A-3 962 074 décrit, de façon très analogue au brevet américain US-A-3 888 167 cité précédemment, la séparation de l'acide acrylique à partir de solutions aqueuses acides par extraction à l'aide d'un mélange de butanol et d'acrylate de butyle.

L'application de tous ces procédés à la récupération de l'acide acrylique et du sulfate acide de butyle à l'aide de butanol ou de mélanges d'acrylate de butyle et de butanol présente de plus l'inconvénient d'entraîner dans l'extrait une concentration importante d'eau et de sels dissous provenant de la neutralisation préalable des acides. Lors d'un recyclage de l'extrait à l'étape d'estérification, la concentration importante d'eau dans cet extrait augmente le temps de réaction. Par ailleurs, lors de cette étape, dans laquelle on élimine l'eau présente, les sels dissous précipitent dans le milieu réactionnel et risquent de boucher les tuyauteries ou d'éroder la paroi du réacteur.

Pour réduire ces inconvénients, on pourrait utiliser de l'acrylate de butyle, ou un mélange d'acrylate de butyle et de butanol riche en ester, ce qui permettrait de réduire l'entraînement d'eau et de sels dans l'extrait. Malheureusement, le rendement d'extraction du sulfate acide de butyle diminue lorsque le rapport ester/alcool dans le solvant augmente.

Appliqués à un procédé synthèse complet de l'acrylate de butyle catalysé par l'acide sulfurique, tous ces procédés génèrent une quantité importante de sels et ne permettent pas de résoudre le problème d'élimination du catalyseur sulfate acide de butyle, responsable d'une partie importante de la pollution aqueuse rejetée.

L'objectif principal de la présente invention est de proposer un procédé perfectionné pour la fabrication de l'acrylate de butyle, par estérification directe sans solvant, en présence d'acide sulfurique utilisé comme catalyseur, ce procédé devant permettre de réduire de façon économique la pollution organique et les rejets salins dans les eaux éliminées, en prenant soin de :
- séparer entièrement l'acide acrylique présent dans le mélange brut après réaction pour éviter son entraînement dans l'ester pur ;
- récupérer le maximum de cet acide acrylique, en vue de le recycler, en réduisant ses pertes génératrices de pollution organique dans les eaux usées du procédé ;
- réduire au maximum la pollution organique rejetée dans les eaux usées du procédé, causée par présence du sulfate acide de butyle dans le milieu réactionnel ; et
- réduire les quantités d'agents neutralisants, responsables de la formation de sels, en recyclant la phase aqueuse acide de l'étape d'hydrolyse du sulfate acide de butyle à l'étape de régénération de l'acide acrylique préalablement neutralisé.

A cet effet, le procédé de fabrication de l'acrylate de butyle, tel qu'il est défini dans le préambule de la présente description, est, conformément à la présente invention, caractérisé par le fait que :
(a) on hydrolyse le sulfate acide de butyle en acide sulfurique à l'aide d'eau pure et/ou à l'aide de l'eau générée au cours de la réaction ;
(b) on décante le milieu après hydrolyse en :
   - une phase organique contenant l'acrylate de butyle, le butanol et une partie de l'acide acrylique non transformé ; et
   - une phase aqueuse contenant l'acide sulfurique et le reste de l'acide acrylique non transformé ;
(c) on effectue un lavage alcalin de la phase organique en vue de neutraliser l'acide acrylique en acrylate alcalin soluble dans la phase aqueuse, puis un lavage à l'eau de la phase organique neutralisée ;
(d) on régénère l'acide acrylique présent sous forme de sel alcalin dans la phase aqueuse de la première neutralisation basique, par addition de la phase aqueuse acide issue de la décantation de l'étape d'hydrolyse, et, éventuellement, d'un complément d'acide sulfurique ;
(e) on extrait l'acide acrylique ainsi régénéré dans cette phase aqueuse par un solvant choisi parmi le butanol, l'acrylate de butyle ou un mélange d'acrylate de butyle et de butanol ;
(f) on recycle dans le réacteur d'estérification la phase organique obtenue en tête de colonne d'extraction, contenant de l'acide acrylique, et du butanol ou de l'acrylate de butyle ou un mélange de butanol et d'acrylate de butyle ;
(g) on envoie la phase aqueuse récupérée en pied de colonne d'extraction au solvant, éventuellement après neutralisation à la soude, en alimentation d'une colonne de distillation permettant de récupérer le butanol en tête, en vue de le recycler en amont du procédé et d'éliminer les eaux épuisées, pratiquement exemptes de pollution organique.

L'hydrolyse de l'étape (a) peut être réalisée dans un réacteur agité ou un réacteur à piston, auquel on adresse le mélange brut réactionnel obtenu après la fin de la réaction d'estérification, à une température de 50 à 200°C, préférentiellement entre 70 et 150°C, et sous une pression suffisante pour éviter l'ébullition du milieu, en ajoutant de l'eau pure et/ou de l'eau générée par la réaction d'estérification, dans une proportion de 3 à 50% en poids par rapport au mélange brut réactionnel, préférentiellement entre 4 et 20% en poids, pendant un temps de 10 secondes à 2 heures, préférentiellement entre 10 secondes et 1 heure.

Les deux lavages de l'étape (c) peuvent être réalisés dans une colonne de lavage alimentée :
- en fond, par la phase organique issue de l'étape d'hydrolyse ;
- en milieu de colonne, par une solution aqueuse de soude ;
- en tête de colonne, par de l'eau fraîche ou le complément de l'eau de réaction non utilisé pour l'étape d'hydrolyse.

Conformément à un mode de réalisation préféré, on réalise les deux lavages de l'étape (c) en neutralisant par une solution basique la phase organique issue du décanteur, et en adressant la phase neutralisée résultante à un nouveau décanteur, la phase organique issue dudit décanteur étant envoyée au pied d'une colonne de lavage alimentée en tête par de l'eau pure et/ou une partie de l'eau générée au cours de la réaction d'estérification, la phase retirée du pied de ladite colonne de lavage étant adressée à la colonne de distillation précitée.

De manière préférentielle, on conduit l'étape d'extraction (e) dans une colonne d'extraction alimentée en tête par la phase aqueuse acidifiée et, en pied, par le solvant.

Ledit solvant peut être constitué par tout ou partie du butanol nécessaire à la réaction d'estérification, éventuellement complété par tout ou partie d'un mélange constitué principalement d'acrylate de butyle et de butanol, issu de colonnes d'étêtage du brut réactionnel lavé et des eaux usées du procédé. Cependant, ledit solvant peut avantageusement être constitué par de l'acrylate de butyle pur avec, le cas échéant, du butanol, le rapport pondéral acrylate de butyle / butanol étant notamment compris entre 1/0 et 1/0,5, préférentiellement entre 1/0 et 1/0,7. Selon un mode de réalisation particulièrement intéressant, le solvant est constitué par tout ou partie du mélange brut réactionnel lavé, ou par mélange d'acrylate de butyle pur et de tout ou partie des flux obtenus en tête des colonnes d'étêtage du brut réactionnel lavé et des eaux usées du procédé.

Le procédé selon la présente invention peut être conduit en continu ou en discontinu.

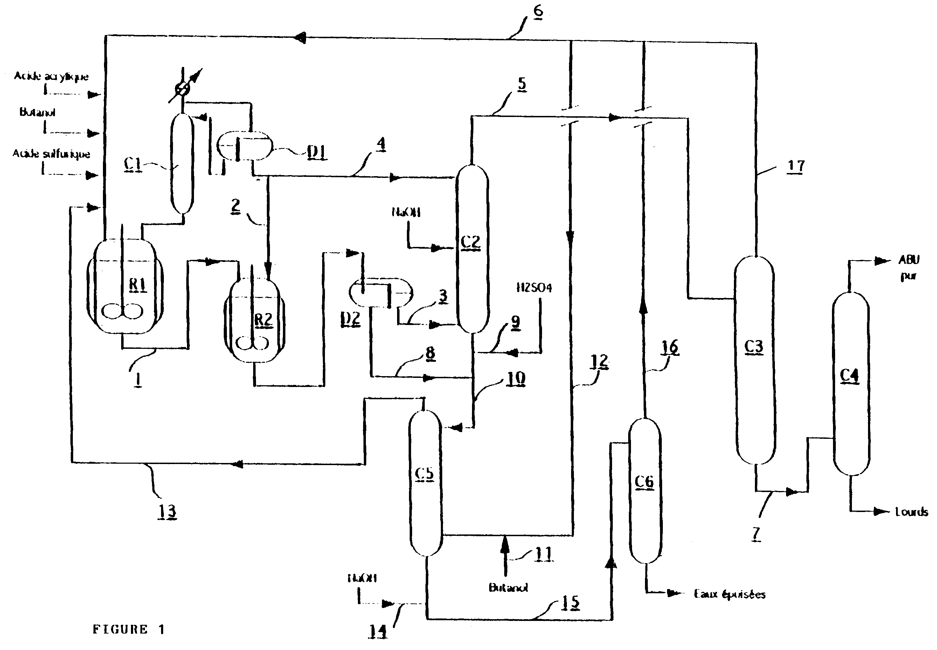

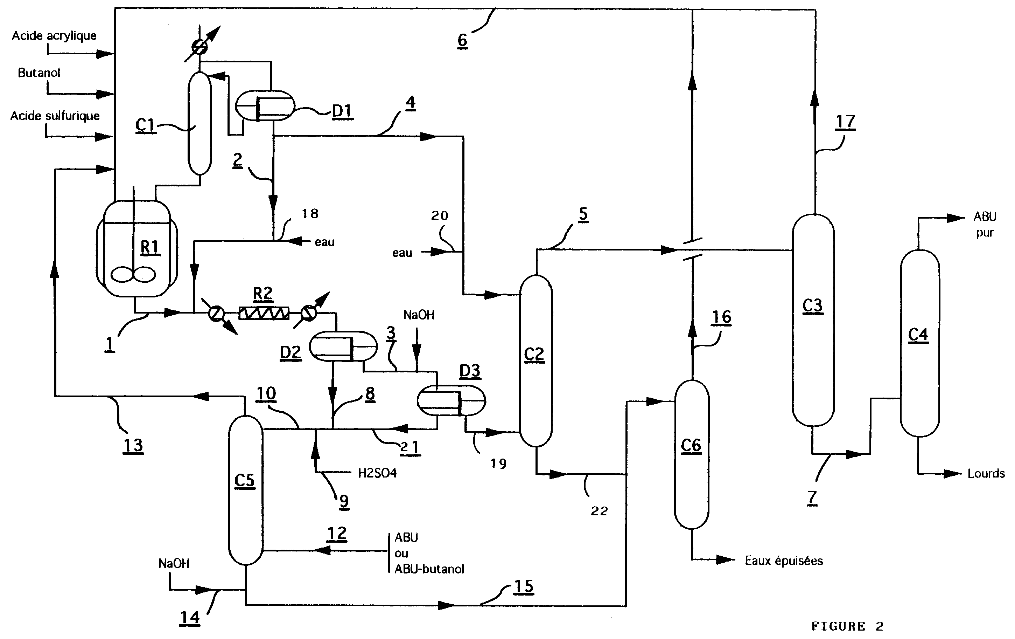

Les Figures 1 et 2 du dessin annexé représentent chacune un schéma du procédé de fabrication de l'acrylate de butyle selon l'invention.

On peut voir sur la Figure 1 que, dans une première étape, qui peut être réalisée en discontinu ou en continu, on envoie dans la partie réactionnelle, qui peut être constituée d'un ou plusieurs réacteurs en série R1 surmontés d'une ou plusieurs colonnes C1, un mélange constitué par l'acide acrylique frais, le butanol frais, l'acide sulfurique utilisé comme catalyseur, ainsi qu'un mélange d'ester et de butanol (6) et un mélange de butanol, ester et acide acrylique (13), récupérés en aval de la réaction dans le procédé décrit. L'eau générée pendant la réaction est distillée pendant la réaction, sous forme d'un mélange azéotropique avec l'alcool et l'ester, puis séparée dans le décanteur D1, où la phase aqueuse est soutirée et la phase organique est renvoyée en tête de la colonne C1.

Le milieu réactionnel est envoyé par la conduite 1 dans un réacteur R2, où l'on effectue l'hydrolyse du sulfate acide de butyle, avec une partie de la phase aqueuse récupérée dans le décanteur D1, amenée via la conduite 2.

Le milieu biphasique obtenu est envoyé dans un décanteur D2, où il est séparé en une phase organique, envoyée par la conduite 3 en pied d'une colonne d'extraction C2. Cette colonne est également alimentée, en tête, par le reste de la phase aqueuse récupérée dans le décanteur D1, via la conduite 4, et en milieu de colonne, par de la soude.

La phase organique, obtenue en tête de colonne C2 est envoyée, via la conduite 5, dans une colonne C3, où l'on récupère en tête (conduite 17) les légers (ABU, butanol, eau) qui seront recyclés à l'étape réactionnelle par la conduite 6. Le pied de la colonne C3 alimente une colonne C4 (flux 7), qui permet de séparer l'ester pur en tête des produits lourds en pied.

La phase aqueuse alcaline, récupérée en pied de la colonne C2, est acidifiée par le flux aqueux acide séparé dans le décanteur D2 (conduite 8), éventuellement complété par de l'acide sulfurique (conduite 9). Le flux acidifié est envoyé, via une conduite 10, en tête d'une colonne d'extraction C5, alimentée en pied par tout ou partie du butanol frais nécessaire à l'estérification (conduite 11) et tout ou partie du flux 12 récupéré en tête des colonnes d'étêtage.

En sortie de cette colonne C5, la phase organique récupérée en tête est recyclée, via la conduite 13, à l'alimentation de l'étape réactionnelle, et la phase aqueuse obtenue en pied est envoyée, après neutralisation des acides par la soude (en 14), dans une colonne de distillation C6 (flux 15).

Cette colonne C6 permet de récupérer, en tête, les composés organiques légers (principalement le butanol) pour les recycler en amont du procédé, via la conduite 16, et de rejeter, en pied, un flux aqueux débarrassé d'une partie importante de sa pollution organique.

Si l'on se réfère maintenant à la Figure 2, on peut voir que l'on a représenté un schéma réactionnel de fabrication d'acrylate de butyle qui diffère de celui de la Figure 1, par les trois point suivants :
- L'hydrolyse du sulfate acide de butyle est conduite dans un réacteur à piston R2 avec de l'eau pure 18 et/ou une partie de la phase aqueuse récupérée dans le décanteur D1, amenée via la conduite 2.
- La phase organique 3, issue du décanteur D2, est neutralisée par une solution basique (NaOH) et le milieu biphasique est séparé dans un nouveau décanteur D3. La phase organique 19 du décanteur D3 est envoyée au pied d'une colonne de lavage C2 alimentée en tête par de l'eau pure 20 et/ou le reste de la phase aqueuse récupérée dans le décanteur D1, via la conduite 4. La phase 22 soutirée en pied de la colonne C2 est adressée en alimentation de la colonne C6.
- La phase aqueuse alcaline, récupérée dans le décanteur D3 (flux 21), est acidifiée par le flux aqueux acide séparé dans le décanteur D2 (conduite 8), éventuellement complété par de l'acide sulfurique (conduite 9). Le flux acidifié est envoyé, via une conduite 10, en tête d'une colonne d'extraction C5, alimentée en pied (conduite 12) par de l'acrylate de butyle pur ou un mélange d'acrylate de butyle et de butanol, constitué par exemple par une partie du flux 5 obtenu en tête de colonne C2, ou par mélange d'acrylate de butyle pur et de tout ou partie des flux 16 et/ou 17 obtenus en tête des colonnes C6 et C3.

Pour illustrer encore l'objet de la présente invention, on va en décrire ci-après, avec référence à la Figure 1, un exemple de mise en oeuvre (Exemple 1) et un exemple comparatif (Exemple 2), et avec référence à la Figure 2, un exemple de mise en oeuvre (Exemple 3), ainsi qu'un exemple décrivant spécifiquement les conditions de mise en oeuvre de l'étape d'hydrolyse du sulfate acide de butyle (Exemple 4).

Dans ces exemples, tous les pourcentages sont exprimés en poids, sauf indication contraire ; ABU désigne l'acrylate de butyle, et SABU désigne le sulfate acide de butyle (BuSO₄H). Les rapports indiqués dans les exemples sont des rapports pondéraux.

### Exemple 1 :

Dans un réacteur d'une capacité de 1 litre, on fait réagir, pendant une durée de 2 heures 10, à une température de 80 - 100°C, 360 g d'acide acrylique, 481 g de butanol, 64 g d'ABU et 13 g d'acide sulfurique. Au fur et à mesure de sa formation, on distille l'eau générée (92 g) dans la colonne surmontant le réacteur, sous forme d'un mélange azéotropique avec le butanol et l'ABU, qui est séparé dans un décanteur en une phase organique recyclée en tête de la colonne et une phase aqueuse qui est soutirée. A l'issue de l'étape réactionnelle, le milieu est composé de 642 g d'ABU, 113 g de butanol, 18 g d'acide acrylique, 20 g de catalyseur, sous forme de SABU.

Ce brut est additionné de 55 g de la phase aqueuse récupérée pendant la réaction et on réalise l'hydrolyse du SABU en acide sulfurique et butanol, en discontinu, à 80°C, pendant 30 minutes. A l'issue de cette étape, le mélange est séparé en une phase aqueuse (72 g) contenant l'acide sulfurique (12,1 g), ainsi que 0,6 g de sulfate de butyle non transformé et 3,6 g d'acide acrylique et une phase organique (804 g) contenant 0,5 g de sulfate de butyle et 14,4 g d'acide acrylique. Le rendement d'hydrolyse est de 95%.

La phase organique est neutralisée par 107 g d'une solution aqueuse de soude 2 N, soit un excès de 5% par rapport à la somme des acidités présentes. Après décantation, la phase organique est exempte d'acidité. Elle est lavée par un mélange constitué par le reste de la phase aqueuse récupérée pendant la réaction, soit 37 g, auxquels on ajoute 100 g d'eau fraîche.

La phase aqueuse alcaline (121 g), obtenue après neutralisation à la soude, est réacidifiée pour régénérer l'acide acrylique, en lui ajoutant un mélange constitué par la phase aqueuse acide (72 g) obtenue après hydrolyse du SABU. Dans ces conditions, l'excès d'acidité par rapport à la stoechiométrie nécessaire pour déplacer les acides (acide acrylique, traces de sulfate de butyle) de leurs sels est de 17%. Il n'est par conséquent pas nécessaire de rajouter de l'acide sulfurique frais. Après agitation de 5 minutes, on obtient une solution aqueuse (192 g) contenant 17,8 g d'acide acrylique, 1 g de sulfate acide de butyle et 1,8 g d'acide sulfurique, ainsi que 15 g de sulfate de sodium.

On procède ensuite à une extraction de l'acide acrylique et du SABU par un mélange constitué de 481 g de butanol et 64 g d'ABU, qui représente l'ensemble des flux d'alcool et d'ester introduits à l'étape d'estérification (butanol frais et mélange butanol et ABU récupéré dans la colonne d'élimination des légers). Dans ces conditions, le rapport d'extraction entre la phase organique et la phase aqueuse est de 2,8.

On récupère une phase organique contenant 17 g d'acide acrylique et 0,7 g de SABU, et une phase aqueuse contenant 1 g d'acide acrylique, 0,3 g de SABU et 1,8 g d'acide sulfurique libre, ainsi que la totalité du sulfate de sodium présent préalablement dans la phase aqueuse acide. Les rendements d'extraction de l'acide acrylique et du SABU sont respectivement de 94% et 70%.

On neutralise l'acidité présente dans la phase aqueuse issue de l'étape d'extraction en ajoutant 27 g de soude 2N. Cette étape génère 4 g de sel sous forme de sulfate de sodium.

Pour 1000 g d'ABU obtenu à l'issue de la réaction d'estérification, on calcule que les rejets de pollution organique dans l'eau, directement liés à la présence d'acide acrylique et de sulfate de butyle représentent une DCO globale de 2,6 g O₂, et que les rejets salins représentent 30 g sous forme de sulfate de sodium.

### Exemple 2 (Exemple Comparatif) :

A l'issue d'une réaction d'estérification conduite dans des conditions identiques à l'Exemple 1, on obtient un milieu réactionnel contenant 638 g d'ABU, 116 g de butanol, 20 g d'acide acrylique et 19 g de SABU.

On procède de la même façon que celle décrite à l'Exemple 1, hormis le fait qu'on n'hydrolyse pas le SABU présent.

Après l'étape de neutralisation, réalisée par ajout de 210 g de soude 2N, la totalité de l'acide acrylique et du SABU sont présents dans la phase aqueuse, sous la forme de sel de sodium.

On réacidifie cette solution aqueuse avec 23,7 g d'acide sulfurique, de façon à régénérer la totalité de l'acide acrylique et du SABU à partir de leurs sels de sodium, en utilisant un excès d'acide de 15% par rapport à la stoechiométrie.

On réalise l'extraction de l'acide acrylique et du SABU dans cette solution aqueuse (248 g), avec les mêmes quantités de butanol (481 g) et d'ABU (64 g) que celles utilisées à l'Exemple 1. Le rapport d'extraction entre le solvant organique et la phase aqueuse est de 2,2.

Après extraction, on récupère une phase organique contenant 18,5 g d'acide acrylique et 12,4 g de SABU, et une phase aqueuse contenant 1 g d'acide acrylique, 6,5 g de SABU et 2,9 g d'acide sulfurique.

La phase aqueuse ainsi obtenue est neutralisée par 60 g de soude 2N (excès de 5%). Cette étape de neutralisation génère 11 g de sel sous forme de sulfate de sodium.

Pour 1000 g d'ABU obtenu à l'issue de la réaction d'estérification, on calcule que les rejets de pollution organique dans l'eau, directement liés à la présence d'acide acrylique et de sulfate de butyle représentent une DCO globale de 14,8 g O₂, soit 5,7 fois plus qu'à l'Exemple 1, et que les rejets salins représentent 65 g sous forme de sulfate de sodium, soit 2,1 fois plus que dans les conditions de l'Exemple 1.

### Exemple 3 :

Dans un réacteur agité, surmonté d'une colonne de distillation équipée d'un décanteur en tête, chauffé à l'aide d'un chauffe réacteur électrique, on fait réagir sous pression réduite, pendant une durée de 2h20, à une température de 80-100°C, un mélange composé d'acide acrylique et de butanol dans un rapport molaire alcool / acide de 1,3/1, de 7% d'ABU (pour simuler le recyclage des légers récupérés en tête de colonne d'étêtage) et de 1,1% d'acide sulfurique à 95%. Au fur et à mesure de sa formation, on distille l'eau générée par la réaction dans la colonne surmontant le réacteur, sous forme d'un mélange azéotropique avec le butanol et l'ABU, qui est séparé dans le décanteur en une phase organique recyclée en tête de la colonne et une phase aqueuse qui est soutirée. A l'issue de l'étape réactionnelle, le milieu est composé d'ABU (82%), de butanol(13%), d'acide acrylique (2,25%), de catalyseur sous forme de SABU (1,8%).

Le brut ainsi obtenu est envoyé en continu (1265 g/h) en même temps qu'une partie de la phase aqueuse récupérée dans le décanteur pendant l'estérification (95 g/h), dans un réacteur constitué d'une conduite en Téflon de diamètre 6 mm et de longueur 3,2 m, remplie de billes de carborundum (carbure de silicium) pour assurer le contact des réactifs. Le réacteur est plongé dans un bain d'huile maintenu à 130°C sous une pression de 2 bars. Le temps de séjour du mélange réactionnel est de 2 minutes. A l'issue de cette étape, le mélange est séparé en une phase aqueuse (91 g/h) contenant 17,2% d'acide sulfurique régénéré et une phase organique (1277 g/h) contenant 0,03% de sulfate de butyle et 2,6% d'acide acrylique ; le rendement d'hydrolyse du SABU est de 98,4% et le rendement d'hydrolyse de l'ABU est limité à 0,3%.

La phase organique est neutralisée par une solution aqueuse de soude 2N, avec un excès de 10% par rapport à la somme des acidités présentes. Après décantation, la phase organique est exempte d'acidité.

La phase aqueuse alcaline obtenue après neutralisation à la soude (soit 1 partie), est mélangée avec la phase aqueuse acide obtenue après hydrolyse du SABU (soit 0,25 partie), et on ajoute à ce mélange de l'acide sulfurique à 95% (soit 0,17 partie). Dans ces conditions, l'excès d'acidité par rapport à la stoechiométrie nécessaire pour déplacer les acides (acide acrylique, traces de sulfate de butyle) de leurs sels est de 10%. Après agitation de 5 mn, on obtient une solution aqueuse contenant 10,2% d'acide acrylique.

On procède ensuite à une extraction de l'acide acrylique par un mélange représentatif de la composition du flux obtenu en tête de colonne de lavage à l'eau (mélange ABU/butanol 9/1), dans un rapport d'extraction entre la phase organique et la phase aqueuse de 0,6/1. L'extraction est réalisée en continu dans une batterie de 4 mélangeurs - décanteurs en série, représentant chacun un étage d'extraction réel, où les phases aqueuse et organique se rencontrent à contre-courant. Ce système représente fidèlement une colonne d'extraction classique comptant 4 étages théoriques.

A la sortie de la batterie d'extraction, on récupère d'une part une phase organique contenant 12% d'acide acrylique, 3,8% d'eau et seulement 13 ppm de sels exprimé en sulfate de sodium, d'autre part une phase aqueuse contenant moins de 0,1% d'acide acrylique, ainsi que la totalité des sels présents initialement dans la phase aqueuse acide. Le rendement d'extraction de l'acide acrylique dans le solvant est de 98%. La teneur en sels dans la phase organique extraite (13 ppm) est compatible avec un recyclage sans lavage de cette phase à l'étape de réaction. En effet, on a pu déterminer que la solubilité du sulfate de sodium dans le milieu réactionnel déshydraté de la réaction d'estérification est de 120 ppm ; il n'y a donc pas de relargage de sels pendant l'estérification. Toutefois, nous avons testé un lavage à l'eau pour éliminer les dernières traces de sels.

Selon une variante des conditions d'extraction décrites ci-dessus, on réalise cette extraction sur 3 étages et un lavage à l'eau de la phase organique extraite sur le 4^{ème} étage. Les mêmes flux aqueux et organiques sont envoyés à contre-courant sur les 3 premiers étages de la batterie, la phase organique sortant du 3^{ème} étage est envoyée dans le 4^{ème} étage, dans lequel on introduit à contre-courant de l'eau pure. Les flux alimentant la batterie sont dans un rapport phase aqueuse acidifiée/solvant/eau pure de 1/0,6/0,1. Dans ce cas, le rendement d'extraction de l'acide acrylique dans la phase organique extraite est de 94% et la teneur en sels, exprimée en sulfate de sodium, est de 0,5 ppm.

Selon une deuxième variante des conditions d'extraction, on effectue cette extraction sur les 4 étages de la batterie de mélangeurs-décanteurs, en envoyant à contre-courant d'une part la phase aqueuse à extraire et d'autre part de l'ABU pur, dans un rapport solvant/phase aqueuse de 0,6/1. Le rendement d'extraction de l'acide sulfurique est de 93%, la concentration en eau dans la phase organique extraite est de 2,9% et celle des sels est inférieure à 0,2 ppm.

Les rejets salins, récupérés intégralement dans l'eau strippée, représentent, d'après le calcul, 40 g pour 1000 g d'ABU purifié.

### Exemple 4

Dans cet exemple, on fait varier en mode continu les conditions d'hydrolyse du SABU dans un brut réactionnel de synthèse d'ABU, et on mesure les rendements d'hydrolyse du SABU (réaction désirée) et de l'ABU (réaction parasite indésirable).

Le mélange brut obtenu en fin de réaction d'estérification d'acide acrylique par le butanol est composé de 83% d'ABU, 10,5% de butanol, 0,2% d'acide acrylique et 1,7% de SABU.

La réaction est faite dans divers types de réacteurs alimentés en continu :
- réacteur agité de capacité 0,5 litre, chauffé par double-enveloppe externe à circulation d'huile
- réacteur tubulaire de diamètre 6 mm, rempli ou non de billes de carborundum (carbure de silicium)
- réacteur tubulaire de diamètre 1,5 mm, non rempli.

Les résultats des divers essais sont résumés dans le Tableau ci-dessous, dans lequel on désigne par R1 le rendement d'hydrolyse du SABU et par R2 le rendement d'hydrolyse de l'ABU.

| Type de réacteur | Réacteur agité | | | | | | Tube rempli | | | Tube vide | |
|---|---|---|---|---|---|---|---|---|---|---|---|
| Diamètre tube | | | | | | | 6 mm | | | 6mm | 1,5mm |
| Température (°C) | 60 | 80 | | | 95 | 105 | 115 | 130 | | 130 | 145 |
| Pression absolue (bars) | 1 | 1 | 1 | 1 | 1 | 1 | 1,5 | 2 | 2 | 2 | 2,6 |
| H₂O/brut | 7 | 7 | 14 | 7 | 7 | 7 | 7 | 7 | 3 | 7 | 7 |
| Temps de séjour (mn) | 30 | 30 | 30 | 60 | 15 | 20 | 2 | 1 | 1 | 2 | 20sec |
| Rendement R1 (%) | 15 | 86 | 85 | 89,5 | 90 | 98,5 | 98,4 | 98,5 | 86 | 97,1 | 95,7 |
| Rendement R2 (%) | 0,4 | 0,8 | 0,9 | 1,9 | 0,8 | 1 | 0,3 | 0,4 | 1,3 | 0,8 | 0,3 |

## Revendications

1. Procédé de fabrication de l'acrylate de butyle par estérification directe de l'acide acrylique par le butanol, ladite estérification étant catalysée par l'acide sulfurique, l'eau de réaction étant éliminée, pendant tout ou partie de la réaction, sous la forme d'un mélange azéotropique avec le butanol et l'acrylate de butyle, le mélange réactionnel brut obtenu comprenant de l'acrylate de butyle, du butanol, de l'acide acrylique, du sulfate acide de butyle et des traces d'acide sulfurique, caractérisé par le fait que :
(a) on hydrolyse (en R2) le sulfate acide de butyle en acide sulfurique à l'aide d'eau pure (18) ou à l'aide de l'eau (2) générée au cours de la réaction ;
(b) on décante (en D2) le milieu après hydrolyse en :
- une phase organique contenant l'acrylate de butyle, le butanol et une partie de l'acide acrylique non transformé ; et
- une phase aqueuse contenant l'acide sulfurique et le reste de l'acide acrylique non transformé ;
(c) on effectue un lavage alcalin de la phase organique en vue de neutraliser l'acide acrylique en acrylate alcalin soluble dans la phase aqueuse, puis un lavage à l'eau de la phase organique neutralisée ;
(d) on régénère l'acide acrylique présent sous forme de sel alcalin dans la phase aqueuse de la première neutralisation basique, par addition de la phase aqueuse acide (8) issue de la décantation (en D2) de l'étape d'hydrolyse, et, éventuellement, d'un complément d'acide sulfurique (9) ;
(e) on extrait (en C5) l'acide acrylique (10) ainsi régénéré dans cette phase aqueuse par un solvant choisi parmi le butanol, l'acrylate de butyle ou un mélange d'acrylate de butyle et de butanol ;
(f) on recycle dans le réacteur d'estérification (R1) la phase organique (13) obtenue en tête de colonne d'extraction (C5), contenant de l'acide acrylique et du butanol ou de l'acrylate de butyle ou un mélange de butanol et d'acrylate de butyle ;
(g) on envoie la phase aqueuse (15) récupérée en pied de colonne (C5) d'extraction au solvant, éventuellement après neutralisation à la soude (en 14), en alimentation d'une colonne de distillation (C6) permettant de récupérer le butanol (16) en tête, en vue de le recycler en amont du procédé et d'éliminer les eaux épuisées, pratiquement exemptes de pollution organique.

2. Procédé selon la revendication 1, caractérisé par le fait qu'on réalise l'hydrolyse de l'étape (a) dans un réacteur agité ou un réacteur à piston (R2), auquel on adresse le mélange brut réactionnel obtenu après la fin de la réaction d'estérification, à une température de 50 à 200°C, et sous une pression suffisante pour éviter l'ébullition du milieu, en ajoutant de l'eau pure (18) et/ou de l'eau (2) générée par la réaction d'estérification, dans une proportion de 3 à 50% en poids par rapport au mélange brut réactionnel, pendant un temps de 10 secondes à 2 heures.

3. Procédé selon la revendication 2, caractérisé par le fait qu'on conduit l'hydrolyse à une température de 70 à 150°C, en ajoutant de l'eau pure (18) et/ou de l'eau (2) générée par la réaction d'estérification dans une proportion de 4 à 20% en poids par rapport au mélange brut réactionnel, pendant un temps de 10 secondes à 1 heure.

4. Procédé selon l'une des revendications 1 à 3, caractérisé par le fait que l'on réalise les deux lavages de l'étape (c) dans une colonne de lavage (C2) alimentée :
- en fond, par la phase organique (3) issue de l'étape d'hydrolyse ;
- en milieu de colonne, par une solution aqueuse de soude;
- en tête de colonne, par de l'eau fraîche ou le complément de l'eau de réaction (4) non utilisé pour l'étape d'hydrolyse.

5. Procédé selon l'une des revendications 1 à 3, caractérisé par le fait que l'on réalise les deux lavages de l'étape (c) en neutralisant par une solution basique la phase organique (3) issue du décanteur (D2), et en adressant la phase neutralisée résultante à un nouveau décanteur (D3), la phase organique (19) issue dudit décanteur (D3) étant envoyée au pied d'une colonne de lavage (C2) alimentée en tête par de l'eau pure (20) et/ou une partie (4) de l'eau générée au cours de la réaction d'estérification, la phase (22) retirée du pied de ladite colonne de lavage (C2) étant adressée à la colonne de distillation (C6).

6. Procédé selon l'une des revendications 1 à 5, caractérisé par le fait que l'on conduit l'étape d'extraction (e) dans une colonne d'extraction (C5) alimentée en tête par la phase aqueuse acidifiée (10) et, en pied, par le solvant (11 ; 12).

7. Procédé selon l'une des revendications 1 à 6, caractérisé par le fait que le solvant de l'étape (e) est constitué par tout ou partie du butanol (11) nécessaire à la réaction d'estérification, éventuellement complété par tout ou partie d'un mélange (12) constitué principalement d'acrylate de butyle et de butanol, issu de colonnes d'étêtage du brut réactionnel lavé et des eaux usées du procédé.

8. Procédé selon l'une des revendications 1 à 6, caractérisé par le fait que le solvant (12) de l'étape (e) est constitué par de l'acrylate de butyle pur avec, le cas échéant, du butanol, le rapport pondéral acrylate de butyle / butanol étant notamment compris entre 1/0 et 1/0,5.

9. Procédé selon la revendication 8, caractérisé par le fait que le solvant est constitué par tout ou partie du mélange brut réactionnel lavé (5), ou par mélange d'acrylate de butyle pur et de tout ou partie des flux (16) et/ou (17) obtenus en tête des colonnes d'étêtage du brut réactionnel lavé et des eaux usées du procédé.

10. Procédé selon l'une des revendications 1 à 9, caractérisé par le fait qu'il est conduit en continu.

11. Procédé selon l'une des revendications 1 à 9, caractérisé par le fait qu'il est conduit en discontinu.

## Claims

1. Process for the manufacture of butyl acrylate by direct esterification of acrylic acid by butanol, the said esterification being catalysed by sulphuric acid, the water of reaction being removed, during all or part of the reaction, in the form of an azeotropic mixture with butanol and butyl acrylate, the crude reaction mixture obtained comprising butyl acrylate, butanol, acrylic acid, butyl hydrogen sulphate and traces of sulphuric acid, characterized in that:
(a) butyl hydrogen sulphate is hydrolysed (in R2) to sulphuric acid using pure water (18) or using water (2) generated during the reaction;
(b) the medium, after hydrolysis, is separated by settling (in D2) into:
- an organic phase containing butyl acrylate, butanol and a part of the unconverted acrylic acid; and
- an aqueous phase containing sulphuric acid and the remainder of the unconverted acrylic acid;
(c) the organic phase is washed under alkaline conditions for the purpose of neutralizing the acrylic acid to alkaline acrylate which is soluble in the aqueous phase and the neutralized organic phase is then washed with water;
(d) the acrylic acid present in the alkaline salt form in the aqueous phase of the first basic neutralization is regenerated by addition of the acidic aqueous phase (8) resulting from the separation by settling (in D2) of the hydrolysis stage and, optionally, of a complement of sulphuric acid (9);
(e) the acrylic acid (10) thus regenerated in this aqueous phase is extracted (in C5) by a solvent chosen from butanol, butyl acrylate or a mixture of butyl acrylate and butanol;
(f) the organic phase (13) obtained at the head of the extraction column (C5), containing acrylic acid and butanol or butyl acrylate or a mixture of butanol and butyl acrylate, is recycled in the esterification reactor (R1);
(g) the aqueous phase (15), recovered at the foot of the solvent extraction column (C5), is conveyed, optionally after neutralization with sodium hydroxide (in 14), as feed to a distillation column (C6) which makes it possible to recover butanol (16) at the head, for the purpose of recycling it upstream in the process, and to discard the spent aqueous liquors, which are virtually free from organic pollution.

2. Process according to Claim 1, characterized in that the hydrolysis in stage (a) is carried out in a stirred reactor or a plug flow reactor (R2), to which is sent the crude reaction mixture obtained after the end of the esterification reaction, at a temperature of 50 to 200°C, and at a pressure which is sufficient to prevent boiling of the medium, by adding pure water (18) and/or water (2) generated by the esterification reaction, in a proportion of 3 to 50% by weight with respect to the crude reaction mixture, for a time from 10 seconds to 2 hours.

3. Process according to Claim 2, characterized in that the hydrolysis is carried out at a temperature of 70 to 150°C, by adding pure water (18) and/or water (2) generated by the esterification reaction in a proportion of 4 to 20% by weight with respect to the crude reaction mixture, for a time of 10 seconds to 1 hour.

4. Process according to one of Claims 1 to 3, characterized in that the two washing operations of stage (c) are carried out in a washing column (C2) supplied:
- at the bottom, with the organic phase (3) resulting from the hydrolysis stage;
- in the middle of the column, with an aqueous sodium hydroxide solution;
- at the head of the column, with fresh water or the remainder of the water of reaction (4) unused in the hydrolysis stage.

5. Process according to one of Claims 1 to 3, characterized in that the two washing operations of stage (c) are carried out by neutralizing the organic phase (3) resulting from the decanter (D2) with a basic solution and by sending the resulting neutralized phase to a new decanter (D3), the organic phase (19) resulting from the said decanter (D3) being conveyed to the foot of a washing column (C2) supplied at the head with pure water (20) and/or a portion (4) of the water generated in the esterification reaction, the phase (22) drawn off from the foot of the said washing column (C2) being sent to the distillation column (C6).

6. Process according to one of Claims 1 to 5, characterized in that the extraction stage (e) is carried out in an extraction column (C5) supplied at the head with the acidified aqueous phase (10), and, at the foot, with the solvent (11; 12).

7. Process according to one of Claims 1 to 6, characterized in that the solvent of stage (e) comprises all or part of the butanol (11) necessary for the esterification reaction, optionally completed by all or part of a mixture (12) mainly comprising butyl acrylate and butanol, resulting from columns for topping the washed crude reaction mixture and the aqueous effluents of the process.

8. Process according to one of Claims 1 to 6, characterized in that the solvent (12) of stage (e) comprises pure butyl acrylate with, if appropriate, butanol, the butyl acrylate/butanol ratio by weight being in particular between 1/0 and 1/0.5.

9. Process according to Claim 8, characterized in that the solvent is composed of all or part of the washed crude reaction mixture (5) or is composed of a mixture of pure butyl acrylate and all or part of the flows (16) and/or (17) obtained at the head of the columns for topping the washed crude reaction mixture and the aqueous effluents of the process.

10. Process according to one of Claims 1 to 9, characterized in that it is carried out continuously.

11. Process according to one of Claims 1 to 9, characterized in that it is carried out non-continuously.

## Patentansprüche

1. Verfahren zur Herstellung von Butylacrylat durch direkte Veresterung von Acrylsäure mit Butanol, wobei die Veresterung durch Schwefelsäure katalysiert wird, das Wasser aus der Reaktion während der gesamten Reaktion oder während eines Teils der Reaktion in Form einer azeotropen Mischung mit Butanol und Butylacrylat entfernt wird und die erhaltene Reaktionsrohmischung Butylacrylat, Butanol, Acrylsäure, saures Butylsulfat (Butylbisulfat, Butylhydrogensulfat) und Spuren von Schwefelsäure enthält, dadurch gekennzeichnet, daß:
(a) man (in R2) Butylhydrogensulfat mit Hilfe von reinem Wasser (18) oder mit Hilfe von während der Reaktion generiertem Wasser (2) zu Schwefelsäure hydrolysiert;
(b) man (in D2) das Milieu nach der Hydrolyse dekantiert:
- in eine organische Phase, die das Butylacrylat, das Butanol und einen Teil der nicht umgesetzten Acrylsäure enthält; und
- in eine wäßrige Phase, die die Schwefelsäure und die restliche, nicht umgesetzte Acrylsäure enthält;
(c) man die organische Phase einer alkalischen Wäsche unterzieht, um die Acrylsäure zu in der wäßrigen Phase löslichem Alkaliacrylat zu neutralisieren, dann die neutralisierte organische Phase mit Wasser wäscht;
(d) man die Acrylsäure, die in der wäßrigen Phase aus der ersten basischen Neutralisation in Form eines Alkalisalzes vorliegt, durch Zugabe der sauren wäßrigen Phase (8), die aus dem Dekantiervorgang (in D2) des Hydrolyseschrittes stammt, und gegebenenfalls durch Zugabe zusätzlicher Schwefelsäure (9) regeneriert;
(e) man (in C5) die auf diese Weise in dieser wäßrigen Phase regenerierte Acrylsäure (10) mit einem Lösemittel extrahiert, das ausgewählt ist aus Butanol, Butylacrylat oder einer Mischung von Butylacrylat und Butanol;
(f) man in dem Veresterungsreaktor (R1) die am Kopf der Extraktionskolonne (C5) erhaltene, Acrylsäure enthaltende organische Phase (13) und Butanol oder Butylacrylat oder eine Mischung von Butanol und Butylacrylat recycliert;
(g) man die am Fuß der Kolonne (C5) zur Lösemittelextraktion erhaltene wäßrige Phase (15), gegebenenfalls nach Neutralisation mit Soda (in 14), einer Destillationskolonne (C6) zuführt, die es ermöglicht, am Kopf das Butanol (16) zu isolieren, um es zu Beginn des Verfahrens wiedereinzusetzen, und das verbrauchte Wasser, das praktisch frei von organischen Verunreinigungen ist, zu entfernen.

2. Verfahren nach Anspruch 1, dadurch gekennzeichnet, daß man die Hydrolyse von Verfahrensschritt (a) in einem Rührkessel oder in einem Kolbenreaktor (R2) durchführt, dem man die Reaktionsrohmischung, die nach Beendigung der Veresterungsreaktion erhalten wird, bei einer Temperatur von 50 bis 200°C und bei einem ausreichenden Druck zuführt, um ein Sieden des Milieus zu vermeiden, indem man reines Wasser (18) und/oder durch die Veresterungsreaktion generiertes Wasser (2) in einer Menge von 3 bis 50 Gew.-%, bezogen auf die Reaktionsrohmischung, über eine Dauer von 10 Sekunden bis 2 Stunden hinzugibt.

3. Verfahren nach Anspruch 2, dadurch gekennzeichnet, daß man die Hydrolyse bei einer Temperatur von 70°C bis 150°C durchführt, indem man reines Wasser (18) und/oder durch die Veresterungsreaktion erzeugtes Wasser (2) in einer Menge von 4 Gew.-% bis 20 Gew.-%, bezogen auf die Reaktionsrohmischung, über eine Dauer von 10 Sekunden bis 1 Stunde hinzugibt.

4. Verfahren nach einem der Ansprüche 1 bis 3, dadurch gekennzeichnet, daß man die zwei Waschvorgänge von Verfahrensschritt (c) in einer Kolonne zur Durchführung des Waschvorgangs (C2) durchführt, die gespeist wird:
- am Boden mit einer organischen Phase (3), die aus dem Hydrolyseverfahrensschritt stammt;
- in der Mitte der Kolonne mit einer wäßrigen Sodalösung;
- am Kopf der Kolonne mit frischem Wasser oder zusätzlich mit Wasser aus der Reaktion (4), das für den Verfahrensschritt der Hydrolyse nicht verbraucht worden ist.

5. Verfahren nach einem der Ansprüche 1 bis 3, dadurch gekennzeichnet, daß man die zwei Waschvorgänge von Verfahrensschritt (c) durchführt, indem man mit einer basischen Lösung die organische Phase (3), die aus der Dekantiervorrichtung (D2) stammt, neutralisiert und man die resultierende neutralisierte Phase einer neuen Dekantiervorrichtung (D3) zuführt, wobei die organische Phase (19), die aus der Dekantiervorrichtung (D3) stammt, dem Fuß einer Waschkolonne (C2) zugeführt wird, die am Kopf mit reinem Wasser (20) und/oder einem Teil (4) des während der Veresterungsreaktion generierten Wassers gespeist wird, wobei die Phase (22), die vom Fuß der Waschkolonne (C2) abgezogen wird, der Destillationskolonne (C6) zugeführt wird.

6. Verfahren nach einem der Ansprüche 1 bis 5, dadurch gekennzeichnet, daß man den Extraktionsverfahrensschritt (e) in einer Extraktionskolonne (C5) durchführt, die am Kopf mit einer angesäuerten wäßrigen Phase (10) und am Fuß mit dem Lösemittel (11; 12) gespeist wird.

7. Verfahren nach einem der Ansprüche 1 bis 6, dadurch gekennzeichnet, daß das Lösemittel von Verfahrensschritt (e) ganz oder teilweise aus dem für die Veresterungsreaktion erforderlichen Butanol (11) besteht, das gegebenenfalls ganz oder teilweise mit einer Mischung (12) versetzt wird, die im wesentlichen aus Butylacrylat und Butanol besteht und aus Abstreifkolonnen für das gewaschene Reaktionsrohprodukt und das verbrauchte Wasser aus dem Verfahren stammt.

8. Verfahren nach einem der Ansprüche 1 bis 6, dadurch gekennzeichnet, daß das Lösemittel (12) von Verfahrensschritt (e) aus reinem Butylacrylat, gegebenenfalls mit Butanol, besteht, wobei das Gewichtsverhältnis von Butylacrylat/Butanol insbesondere zwischen 1/0 und 1/0,5 liegt.

9. Verfahren nach Anspruch 8, dadurch gekennzeichnet, daß das Lösemittel ganz oder teilweise aus der gewaschenen Reaktionsrohmischung (5) oder einer Mischung von reinem Butylacrylat und ganz oder teilweise von den Zuflüssen (16) und/oder (17) besteht, die am Kopf der Abstreifkolonnen für das gewaschene Reaktionsrohprodukt und das verbrauchte Wasser aus dem Verfahren erhalten werden.

10. Verfahren nach einem der Ansprüche 1 bis 9, dadurch gekennzeichnet, daß es kontinuierlich durchgeführt wird.

11. Verfahren nach einem der Ansprüche 1 bis 9, dadurch gekennzeichnet, daß es diskontinuierlich durchgeführt wird.
